# EUROPEAN PATENT APPLICATION

(11) **EP 4 745 250 A1**
(43) Date of publication of application: **20.05.2026**
(21) Application number: 25152595.2
(22) Date of filing: 17.01.2025
(51) Int. Cl.: C12Q 1/70

(54) **DETECTION CHIP FOR AFRICAN SWINE FEVER VIRUS AND APPLICATION THEREOF**

(30) Priority: 18.11.2024 CN 202411644783
(71) Applicant: Sunplus Technology Co., Ltd, Hsinchu 300 (TW); Kao, Hsi-Teng, Hsinchu City 300 (TW)
(72) Inventor: KAO, Hsi-Teng, 300 Hsinchu City (TW); CHIOU, Ming-Tang, 900 Pingtung City, Pingtung County (TW); LIN, Wei-Hao, 906 Pingtung County (TW); CHENG, Daisy, 241 New Taipei City (TW); LEE, Yi-Chan, 300 Taoyuan City (TW); JEN, Shu-I, 300 Hsinchu City (TW)
(74) Representative: Wittmann, Günther

(57) **Abstract**

The present invention relates to a biological detection chip for African swine fever virus and its application thereof. The biological detection chip comprises a plurality of transistors connected in parallel, each of which individually includes a substrate layer, a floating gate, a metal connecting channel, an extending gate, and a biological detection layer. The biological detection chip is functionalized through surface modification process to integrate biological probes, enabling the detection of African swine fever virus in test samples.

## Description

### SUBMISSION OF SEQUENCE LISTING AS ASCII TEXT FILE

This application includes an electronically submitted sequence listing in XML format. The XML file contains a sequence listing entitled "P24-0222EPC Sequence Listing.xml" which was created on Jan. 13, 2025 and is 4,328 bytes in size. The sequence listing contained in this XML file is part of the specification and is hereby incorporated by reference herein in its entirety.

### BACKGROUND OF THE INVENTION

### 1. FIELD OF THE INVENTION

The present invention relates to a biological detection chip and its applications, specifically to a biochip for detecting African swine fever virus, a nucleic acid probe, and the associated detection method.

### 2. DESCRIPTION OF THE PRIOR ART

African swine fever virus (ASFV) is a double-stranded DNA virus with high infectivity and has a high fatality rate after infection. ASFV primarily affects pigs and does not infect humans. African swine fever was initially discovered in Africa and has spread globally due to the expansion of global trade and transportation. The virus has caused significant economic losses to the global swine industry. Since there are currently no effective vaccines or treatments available, the focus of epidemic prevention is on detecting the virus and culling infected pigs.

Traditional virus detection methods, such as polymerase chain reaction (PCR), are challenging to operate outside laboratory settings. They require specialized equipment and trained personnel, making it difficult to perform rapid and effective detection directly at pig farms. This limitation often delays outbreak control. Furthermore, PCR detection method is less sensitive. If the virus content is low, it may result in failure to detect infected pigs carrying the pathogen. PCR detection also has a problem of non-specificity.

In summary, the development of effective methods for detecting African swine fever virus that improve the shortcomings of low sensitivity, long detection time, and operational complexity is a critical issue that needs to be solved in the relevant field.

### SUMMARY OF THE INVENTION

In view of the drawbacks of current detection technologies for African swine fever virus, the present invention incorporates biochips into the detection process and provides nucleic acid probes with high accuracy and sensitivity to optimize detection performance and workflow.

Those skilled in the art will recognize, or be able to ascertain using no more than routine experimentation, many equivalents to the specific embodiments of the invention described herein. Such equivalents are intended to be encompassed by the following embodiments.

Embodiment 1. A biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising a plurality of biological probes, wherein the biological probes comprise a nucleotide sequence of SEQ ID NO: 1;
wherein the biological detection layers of the plurality of transistors form a biological detection area on the surface of the biological detection chip.

Embodiment 2. The biological detection chip according to Embodiment 1, wherein the biological detection layer is generated through a surface modification process on the surface of the extending gate.

Embodiment 3. The biological detection chip according to Embodiment 1 or 2, wherein the biological detection area comprises a microfluidic channel or a sample reservoir.

Embodiment 4. The biological detection chip according to any one of Embodiments 1 to 3, wherein the surface modification process is one of 3-aminopropyltrimethoxysilane-Glutaraldehyde (APTES-GA) modification method, 3-aminopropyltrimethoxysilane-N-hydroxysuccinimide (APTES-NHS) modification method, 3-aminopropyltrimethoxysilane-Biotin (APTES-Biotin) modification method, and 3-glycidoxypropyltrimethoxysilane (GPTMS) modification method.

Embodiment 5. The biological detection chip according to any one of Embodiments 1 to 4, wherein the biological detection chip is set on a handheld detector.

Embodiment 6. A method for detecting African swine fever virus, wherein the method utilizes the biological detection chip of the present invention to perform bio-detection on a test sample, comprising the following steps:
providing a biological detection chip of the present invention;
placing a blank sample in the biological detection area, and measuring a gate voltage and a drain current of the plurality of transistors to establish a standard curve;
placing a test sample in the biological detection area for a predetermined time;
washing the biological detection area by the blank sample;
measuring the gate voltage and the drain current of the plurality of transistors to establish a measurement curve; and
comparing the measurement curve with the standard curve to determine whether the test sample contains African swine fever virus.

Embodiment 7. The detection method according to Embodiment 6, wherein the test sample is derived from a pig.

Embodiment 8. The detection method according to Embodiment 6 or 7, wherein the test sample is a liquid sample.

Embodiment 9. The detection method according to Embodiment 8, wherein the liquid sample comprises a whole blood sample, a serum sample, an oral fluid sample or a urine sample.

Embodiment 10. The detection method according to Embodiment 6 or 7, wherein the test sample is a swab sample.

Embodiment 11. The detection method according to Embodiment 10, wherein the swab sample comprises a nasal swab sample, an oral swab sample or a rectal swab sample.

Embodiment 12. A biological probe, comprising a nucleotide sequence of SEQ ID NO: 1, wherein the biological probe is used for detecting African swine fever virus.

Embodiment 13. The biological probe according to Embodiment 12, wherein the nucleotide sequence comprising at least 90% sequence identity to SEQ ID NO: 1, for example, 90%, 91%, 92%, 93%, 94%, 95%, 96%, 97%, 98%, 99%, or 100% sequence identity.

The African swine fever detection chip provided by the present invention offers advantages such as high sensitivity, excellent accuracy, and ease of operation. In some embodiments, the detection chip can be configured as a handheld detection device. By connecting to a mobile phone or computer, data can be transmitted, analyzed, and detection results can be returned. Additionally, the biochip described in the present invention can detect a variety of test samples with straightforward operation, enabling direct testing at pig farms, which facilitates industrial applications. Furthermore, the biological probe designed in the present invention is capable of detecting various African swine fever virus variants while maintaining high sensitivity and accuracy, significantly enhancing its practical utility in the industry.

### BRIEF DESCRIPTION OF THE DRAWINGS

The accompanying drawings illustrate one or more embodiments of the invention and, together with the written description, serve to explain the principles of the invention. Wherever possible, the same reference numbers are used throughout the drawings to refer to the same or like elements of an embodiment.
FIG. 1 is a schematic diagram of the biological detection chip in accordance with one embodiment of the present disclosure.
FIG. 2 is a cross-sectional schematic diagram of the biological detection chip along the dotted line AA' in FIG. 1 in one embodiment of the present disclosure.
FIG. 3 is a schematic diagram illustrating the surface modification process in one embodiment of the present disclosure.
FIG. 4 is a signal quantification diagram for the detection of African swine fever virus (ASFV) using the biological detection chip in Example 2 of the present disclosure.
FIG. 5 is a survival rate chart of pigs in different groups as described in Example 3 of the present disclosure.
FIG. 6A shows the body temperature changes of pigs in the low-virulence virus strain group. FIG. 6B shows the body temperature changes of pigs in the high-virulence virus strain group. FIG. 6C shows the body temperature changes of pigs in the recombinant virus strain group.
FIG. 7A is a scatter plot of the days to first detection of the virus in blood samples from pigs in the low-virulence virus strain group after challenge. FIG. 7B is a scatter plot of the days to first detection of the virus in blood samples from pigs in the high-virulence virus strain group after challenge. FIG. 7C is a scatter plot of the days to first detection of the virus in blood samples from pigs in the recombinant virus strain group after challenge.
FIG. 8A is a scatter plot of the days to first detection of the virus in nasal swab samples from pigs in the low-virulence virus strain group after challenge. FIG. 8B is a scatter plot of the days to first detection of the virus in nasal swab samples from pigs in the high-virulence virus strain group after challenge. FIG. 8C is a scatter plot of the days to first detection of the virus in nasal swab samples from pigs in the recombinant virus strain group after challenge.
FIG. 9A is a scatter plot of the days to first detection of the virus in oral fluid samples from pigs in the low-virulence virus strain group after challenge. FIG. 9B is a scatter plot of the days to first detection of the virus in oral fluid samples from pigs in the high-virulence virus strain group after challenge. FIG. 9C is a scatter plot of the days to first detection of the virus in oral fluid samples from pigs in the recombinant virus strain group after challenge.
FIG. 10A is a scatter plot of the days to first detection of the virus in rectal swab samples from pigs in the low-virulence virus strain group after challenge. FIG. 10B is a scatter plot of the days to first detection of the virus in rectal swab samples from pigs in the high-virulence virus strain group after challenge. FIG. 10C is a scatter plot of the days to first detection of the virus in rectal swab samples from pigs in the recombinant virus strain group after challenge.

### DETAILED DESCRIPTION OF THE PREFERRED EMBODIMENT

The following examples are provided to illustrate the embodiments of the present disclosure. A person skilled in the art will readily understand the advantages and efficacy of the present disclosure based on the teachings revealed in this specification. The present disclosure may also be implemented or applied in other different embodiments. The details provided in this specification may be modified and altered from various perspectives and applications without departing from the scope disclosed herein.

In view of the drawbacks of current detection technologies for African swine fever virus, the present invention incorporates biochips into the detection process and provides nucleic acid probes with high accuracy and sensitivity to optimize detection performance and workflow. The biochip provided by the present invention generates energy changes through the interaction between the identification element and the object to be measured, and converts the energy changes into signals for subsequent detection and analysis. It has the effects of rapid detection, convenient operation, and high sensitivity.

In one aspect, the present invention provides a biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising a plurality of biological probes, wherein the biological probes comprise a nucleotide sequence of SEQ ID NO: 1;
wherein the biological detection layers of the plurality of transistors form a biological detection area on the surface of the biological detection chip.

Preferably and in some embodiments, the biological detection layer is generated through a surface modification process on the surface of the extending gate.

Preferably and in some embodiments, the biological detection layer is generated through a surface modification process on the surface of the extending gate. In some embodiments, the surface modification process is one of 3-aminopropyltrimethoxysilane-Glutaraldehyde (APTES-GA) modification method, 3-aminopropyltrimethoxysilane-N-hydroxysuccinimide (APTES-NHS) modification method, 3-aminopropyltrimethoxysilane-Biotin (APTES-Biotin) modification method, and 3-glycidoxypropyltrimethoxysilane (GPTMS) modification method.

Preferably and in some embodiments, the biological detection area comprises a microfluidic channel or a sample reservoir, which is used to load a test sample.

Preferably and in some embodiments, the biological detection chip is set on a handheld detector.

In another aspect, the present invention provides a method for detecting African swine fever virus, wherein the method utilizes the biological detection chip described in the present invention to perform bio-detection on a test sample, comprising the following steps:
providing a biological detection chip described in the present invention;
placing a blank sample in the biological detection area, and measuring a gate voltage and a drain current of the plurality of transistors to establish a standard curve;
placing a test sample in the biological detection area for a predetermined time;
washing the biological detection area by the blank sample;
measuring the gate voltage and the drain current of the plurality of transistors to establish a measurement curve; and
comparing the measurement curve with the standard curve to determine whether the test sample contains African swine fever virus.

Preferably and in some embodiments, the test sample is derived from a pig.

Preferably and in some embodiments, the test sample is a liquid sample or a swab sample. Preferably and in some embodiments, the liquid sample comprises a whole blood sample, a serum sample, an oral fluid sample or a urine sample. Preferably and in some embodiments, the swab sample comprises a nasal swab sample, an oral swab sample or a rectal swab sample.

In another aspect, the present invention provides a biological probe, comprising a nucleotide sequence of SEQ ID NO: 1, wherein the biological probe is used for detecting African swine fever virus.

Preferably and in some embodiments, the nucleotide sequence comprising at least 90% sequence identity to SEQ ID NO: 1, for example, 90%, 91%, 92%, 93%, 94%, 95%, 96%, 97%, 98%, 99%, or 100% sequence identity.

Unless otherwise defined herein, scientific and technical terms used in connection with the present disclosure shall have the meanings that are commonly understood by those of ordinary skill in the art. The terminology used in this specification is used to describe particular embodiments only and is not intended to limit the invention.

As used herein, the singular form "a", "an", and "the" includes plural references unless indicated otherwise. For example, "an" excipient includes one or more excipients. The term "or" is used interchangeably with the term "and/or" unless the context clearly indicates otherwise.

As used interchangeably herein, "around", "about" and "approximately" shall generally mean plus or minus 5% of the numerical value of the number with which it is being used. Numerical quantities given herein are approximate, meaning that the term "around", "about" or "approximately" can be inferred if not expressly stated.

The phrase "comprising" as used herein is open-ended, indicating that such embodiments may include additional elements. In contrast, the phrase "consisting of" is closed, indicating that such embodiments do not include additional elements (except for trace impurities). The phrase "consisting essentially of" is partially closed, indicating that such embodiments may further comprise elements that do not materially change the basic characteristics of such embodiments.

As used herein, the term "GPTMS modification method" refers to a surface modification process that involves the treatment of 3-glycidoxypropyltrimethoxysilane (GPTMS).

The term "approximate the same distance" as used herein refers to two distances that are either equal or where the second distance is approximately equal to the first distance, meaning the second distance falls within ±10% of the first distance. For example, if the first distance is 1 cm, the second distance that is approximate to the first would be 1 cm or within the range of 0.9 to 1.1 cm.

In order to facilitate the understanding of the technical features, the contents, and the advantages of the present disclosure, and the effectiveness thereof that can be achieved, the present disclosure will be illustrated in detail below through embodiments with reference to the accompanying drawings. On the other hand, the diagrams used herein are merely intended to be schematic and auxiliary to the specification but are not necessary to be true scale and precise configuration after implementing the present disclosure. Thus, it should not be interpreted in accordance with the scale and the configuration of the accompanying drawings to limit the scope of the present disclosure on the practical implementation.

### Structure of Biological Detection Chip

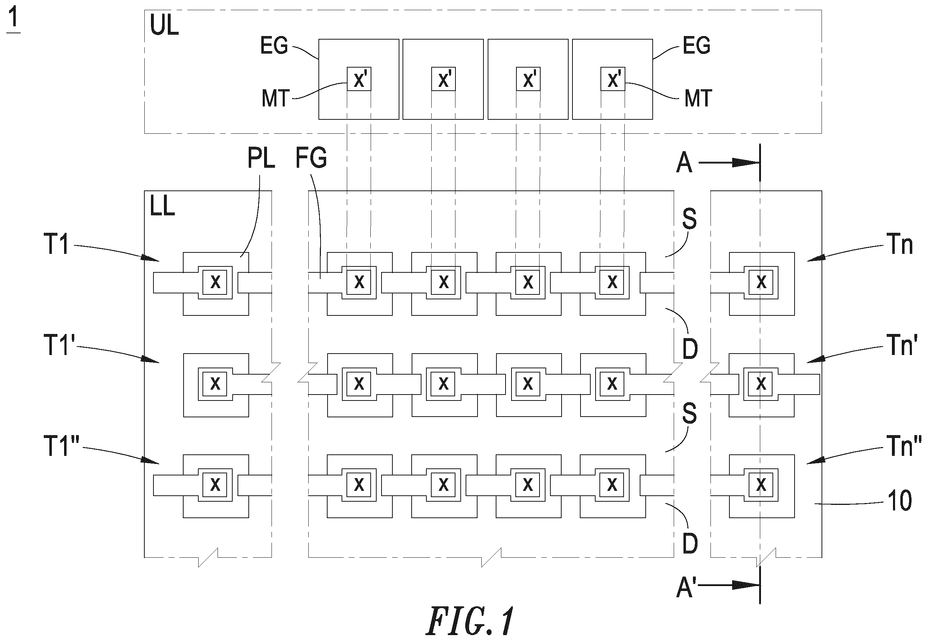

As shown in FIG. 1, which is the schematic diagram of the biological detection chip in accordance with one embodiment of the present disclosure. As shown in FIG. 1, the biological detection chip 1 includes n transistors T1-Tn arranged in parallel. The array has a plurality of rows and a plurality of columns. Each transistor is disposed within one of the rows and one of the columns. The quantities of the transistors can be determined by the demand of biological detection chip 1. The structure of the transistors T1-Tn connected in parallel includes an upper layer UL and a lower layer LL. Take the transistor T1 as an example; the substrate layer 10 contains a shared source S, a shared drain D, and a channel area disposed between the shared source S and the shared drain D. The floating gate FG is disposed on the channel area between the shared source S and the shared drain D. A polysilicon oxide layer PL is disposed under the floating gate FG. An extending metal connector MT is disposed on the floating gate FG at the location of the X block and extends through the floating gate FG to the upper layer UL at the locations of the X' block. The extending gate EG is disposed on the extending metal connector MT. Therefore, the extending gate EG of the upper layer UL is electrically connected to the floating gate FG of the lower layer LL.

In addition, as shown in FIG. 1, the floating gate FG of the transistor T1 and the floating gate FG of the adjacent transistor T1' in the lower row can be respectively close to the same shared drain D at an approximately the same distance, and the floating gate FG of the adjacent transistor T1' in the lower row and the floating gate FG of the adjacent transistors T1" below the lower row can be respectively close to the same shared source S at an approximately the same distance. That is, transistors T1, T1', and T1" are disposed in the same column, and the floating gate FG of transistor T1 and the floating gate FG of transistor T1' have approximately the same distances to the shared drain D between transistors T1 and T1'. Similarly, the floating gate FG of transistor T1' and the floating gate FG of transistor T1" have approximately the same distances to the shared source S between transistors T1' and T1".

In some embodiment, the extending gate EG is a metal plate electrode. The extending gate EG connects to the covered polysilicon oxide layer PL through the extending metal connector MT, so that the extending gate EG of the upper layer UL may electrically connect to the floating gate FG. In other embodiments, the extending gate EG may be a metal electrode in other shapes. For example, the extending gate EG may be a spiral shape electrode. In addition, several rows of the n transistors T1-Tn connected in parallel can be arranged to form a structure with a plurality of detection layers. Between each detection layer, the adjacent detection layers share the shared source S or the shared drain D. When measuring the transistors T1-Tn, the transistors T1-Tn are connected in parallel. The shared source S may connect to the ground and the shared drain D may connect to a current measuring terminal. Based on the voltage applied to the extending gate EG, a sum of the drain current can be measured as detection data for judging the result of the biological detection.

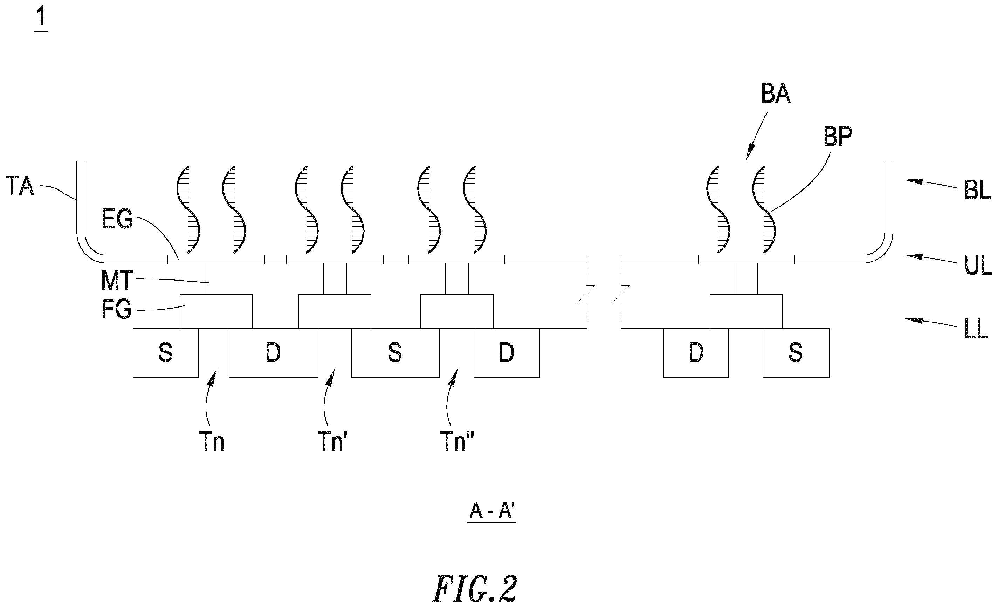

In order to conduct the biological detection, a biological detection layer BL is disposed on the extending gate EG of the transistors T1-Tn. The biological detection layer BL can bind a specific biological target. When a biological detection layer BL binds a specific biological target, the electronic feature of the transistor changes. The measurement of the current is used to determine whether there is a specific biological target. Regarding the biological detection layer BL, FIG. 2 shows an embodiments of the cross-sectional views of the biological detection chip along the dotted line AA' in FIG. 1. As shown in FIG. 2, the biological detection chip 1 includes a plurality of detection columns, and the transistors of the plurality of detection columns all include the structure of the upper layer UL and the lower layer LL, and the biological detection layer BL is disposed on the upper layer UL.

As shown in FIG. 2, take the transistor Tn as an example; the transistor Tn includes the shared source S and the shared drain D. The floating gate FG is disposed on the channel area between the shared source S and the shared drain D. The floating gate FG is electrically connected to the extending gate EG through the extending metal connector MT. As shown in FIG. 2, in one embodiment, each of the floating gate FG of the transistor Tn and the floating gate FG of the adjacent transistor Tn' may cover at least a part of the same shared drain D; each of the floating gate FG of the adjacent transistor Tn' and the floating gate FG of another transistor Tn" which abuts upon the adjacent transistor Tn' may cover at least a part of the same shared source S. In a preferable embodiment, the transistors Tn, Tn', and Tn" are disposed in the same column, and the floating gate FG of transistor Tn and the floating gate FG of transistor Tn' have approximately the same distances overlapping the shared drain D between transistors Tn and Tn'. Similarly, the floating gate FG of transistor Tn' and the floating gate FG of transistor Tn" have approximately the same distances overlapping the shared source S between transistors Tn' and Tn".

The biological detection layer BL is disposed on the extended gate EG, where multiple biological probes BP are immobilized on the surface of the extended gate EG through a surface modification process to facilitate subsequent biological detection.

The biological probes BP used in the present invention are nucleic acid probes. The nucleic acid probes possess the nucleotide sequence shown in SEQ ID NO: 1 and are designed for detecting African swine fever virus. The biological probes BP are immobilized on the biological detection layer BL through biological conjugation. When biological detection is conducted, the biological probes BP bind the biological targets in a sample, and the generated charge may affect the drain current signal detected from the transistor Tn. This allows for determining whether the sample contains African swine fever virus.

The biological detection chip 1 may form a detection layer by several transistors connected in parallel. The plurality of detection layers may further form a detection array, so as to form a plurality of biological detection area BA on the surface of the biological detection chip 1. A sample tank TA may be set at the biological detection layer BL to create an accommodating space, so as to contain biological detection solution in the biological detection area BA. Therefore, the biological target in the biological detection solution can be accommodated within the biological detection areas BAs. The biological detection solution may cover the surface of the biological detection chip 1, so that the biological target and the biological probes BP of the biological detection layer BL can contact and bind to each other. In the present embodiment, the accommodating space is a sample tank TA. However, the accommodating space may be in a different form, such as, but not limited to, a microflow channel disposed at the biological detection layer BL.

In summary, as shown in FIG. 1 and 2, the present invention provides a biological detection chip 1, comprising a plurality of transistors disposed in an array having a plurality of rows and a plurality of columns, each of the plurality of transistors being disposed within one of the plurality of rows and one of the plurality of columns, and each of the plurality of transistors Tn comprising:
a substrate layer 10 comprising a shared source S, a shared drain D, and a channel area 103 disposed between the shared source S and the shared drain D;
a floating gate FG disposed on the channel area 103 and comprising a polysilicon oxide layer PL;
an extending metal connector MT disposed on the floating gate FG;
an extending gate EG disposed on the extending metal connector MT and electrically connected to the floating gate FG through the extending metal connector MT; and
a biological detection layer BL disposed on the extending gate EG and comprising a plurality of biological probes, wherein the biological probes comprise a nucleotide sequence of SEQ ID NO: 1.

In some embodiments, the biological detection layer BL of the multiple transistors Tn forms a biological detection area BA on the surface of the biological detection chip 1.

In the array, the multiple transistors (T1~Tn) in the same row are connected in parallel. Among the multiple transistors (Tn, Tn', Tn", ...) in the same column, the floating gates FG of any two adjacent transistors (e.g., Tn and Tn') are positioned at an approximate distance near the shared source S or shared drain D between the two transistors, or partially cover at least a portion of the shared source S or shared drain D between the two transistors.

The present invention is further illustrated by the following Examples, which in no way should be construed as further limiting. The entire contents of all of the references (including literature references, issued patents, published patent applications, and co-pending patent applications) cited throughout this application are hereby expressly incorporated by reference.

### Example 1 Preparation of African swine fever detection chip

In this embodiment, the surface modification process was performed on the extended gate surface using the GPTMS modification method, immobilizing multiple biological probes with the nucleotide sequence shown in SEQ ID NO: 1 onto the biological detection layer.

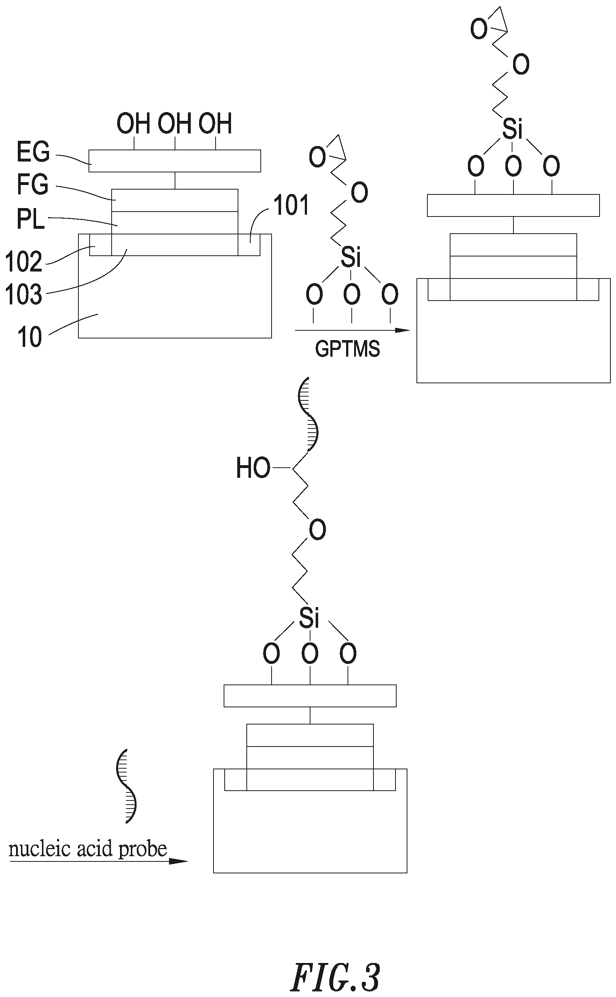

As shown in FIG. 3, which illustrates the GPTMS modification method employed in this embodiment. The steps involved in the GPTMS modification method are described as follows:
A packaged chip was first sonicated in acetone and then sonicated in 95% ethanol, followed by drying at 90-120°C to remove ethanol. The chip was then subjected to oxygen plasma treatment to incorporate hydroxyl groups (-OH) onto the surface of the extending gate.
After that, the chip was immersed in 3-glycidoxypropyltrimethoxysilane (GPTMS) dissolved in absolute ethanol and then sonicated in absolute ethanol. Then, the chip was dried at 90-120°C to remove ethanol and make epoxide group of GPTMS bind to the hydroxylated surface of the extending gate.
Subsequently, multiple biological probes (SEQ ID NO: 1) with amino groups (-NH₂) were conjugated to the epoxide groups in the biological detection layer, so the biological probes were immobilized on the chip.
After treatment with a blocking agent, such as BSA, the chip was washed, dried with nitrogen gas, and placed in a mold for vacuum storage or further use.

### Example 2 Detection effect of African swine fever detection chip

In this embodiment, the African swine fever detection chip of the present invention is tested to explore its detection limit (Limit of Detection, LoD).

### Biological Detection method

In this embodiment, the biological detection method of the biological detection chip as described in the present invention includes the following steps (Step 1-Step 5). The detection process of this embodiment can also be found in the AttoSense FET Biosensor Kit:
Step 1: providing a biological detection chip of the present invention, wherein the preparation method of the biological detection chip is as described in Example 1. In some embodiments, the biological detection chip of the present invention is set on a handheld detector to facilitate subsequent use.
Step 2: placing a blank sample in the biological detection area, and measuring a gate voltage and a drain current of the plurality of transistors to establish a standard line. In some embodiments, 140 µL of blank sample is introduced into the sample reservoir for calibration to establish the standard curve. After calibration, the liquid in the sample reservoir is removed.
Step 3: placing a test sample in the biological detection area for a predetermined time. The test sample solution is placed in the biological detection area. For example, the test sample is placed in the sample tank for a period of time, so that the biological target can bind to the biological probes of the biological detection layer. The reaction time for the test sample is determined by the biological target species. In some embodiment, 140 µL of the test sample is placed in the sample reservoir and react for 15 minutes.
Step 4: washing the biological detection area with the blank sample. To ensure effective binding between the test sample and the biological probes, the biological detection area is washed after the predetermined reacting time, and the test sample is placed again. Repeated washing and placement ensure sufficient binding of the test sample. In some embodiments, 140 µL of blank sample is introduced into the sample reservoir and washed twice. After washing, the liquid in the sample reservoir is removed.
Step 5: measuring the gate voltage and the drain current of the plurality of transistors to establish a measurement line, and comparing the measurement line with the standard line to determine whether the test sample contains the biological target. After the reaction (Step 3) and washing (Step 4), different gate voltages are applied to the extending gate EG again. The output current I1-In of the shared drain D are measured by the current measuring device and the sum of the output current I1-In is converted to a digital signal through the voltage converter. The information of the drain current is output from the output terminal, and the measurement line of the test sample is established accordingly. The measurement line is then compared to the standard line to determine how much the measurement line deviates from the standard line, and to further determine whether the test sample contains the African swine fever virus based on the level of the deviation. When a test sample does not contain African swine fever virus, the biological probes on the detection chip do not bind to anything; there is no change of the gate charge of the chip, so the measurement line does not obviously deviate from the standard line. In contrast, when a test sample contains African swine fever virus, the biological probes on the detection chip bind to the genetic material of African swine fever virus; there is change of the gate charge of the chip, so the measurement line deviates from the standard line. Therefore, if there is no obvious deviation of a measurement line from the standard line, it is determined that the test sample does not contain the biological target. However, if the deviation of a measurement line from the standard line exceeds a predetermined level, it is determined that the test sample contains the biological target.

In some embodiments, during electrical measurements, a voltage ranging from 0V to 2V (0, 0.03, 0.06...2V) or 1.5V to 3V, depending on the biological target to be detected, is applied to the sample tank. There are 63 voltage values in total. The measurement software records the voltage value transmitted from the sensing area to the semiconductor device under each applied voltage. This process generates 63 sets of numerical values, which are then plotted to produce an electrical curve. In addition, the term "chip signal value" as used herein refers to quantifying the signal. This quantification is calculated through a trapezoidal formula, which is employed to calculate the area between the standard curve and the sample curve. The calculation is performed as follows: |((Target2+Target1)*1/2)-((Baseline2+Baseline1)*1/2))|+|((Target3+Target2)*1/2)-((Baseline3+Baseline2)*1/2))|+...+|((Target63+Target62)*1/2)-((Baseline63+Baseline62)*1/2))|. In some embodiments, the chip signal measured by the blank sample is regarded as the background signal. When the chip signal of the test sample exceeds the background signal by two standard deviations, the test sample is judged to contain African swine fever virus and is regarded as a positive sample.

### Experimental materials

The test sample used in the present embodiment is African swine fever virus (ASFV) particles. The sample was lysed using VLL buffer (LabTurbo^{™} Virus Mini Kit, LVN480-200, LabTurbo Biotech Corporation, Taipei, Taiwan) with a reaction time of 15 minutes. The prepared sample was further diluted using 25 mM Tris-HCl buffer. After dilution, the virus concentrations of the test samples are 0 copies/µL (control group), 10 copies/mL, 100 copies/µL and 10,000 copies/µL. These samples were used to evaluate the detection limit of the biological detection chip for African swine fever virus as described in the present invention.

### Results

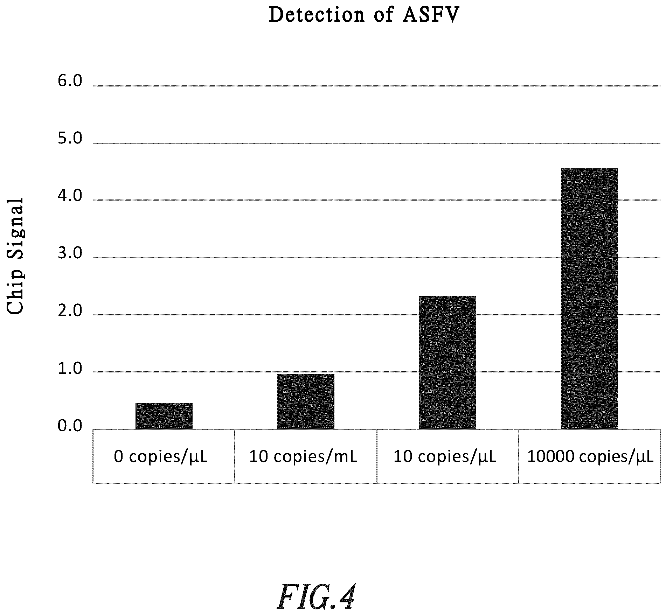

As shown in FIG. 4, the control group with a virus concentration of 0 copies/µL detected a chip signal of approximately 0.45, which is considered the background signal. For virus concentrations of 10 copies/mL, 10 copies/µL, and 10,000 copies/µL, the chip signal detected were 0.96, 2.33, and 4.56, respectively. The results demonstrate that higher virus concentrations produce stronger chip signals, confirming that the biological detection chip described in the present invention is capable of detecting African swine fever virus. Furthermore, a virus concentration of 10 copies/mL already resulted in a chip signal higher than the background signal, indicating that the detection limit can reach at least 10 copies/mL which highlights the high sensitivity of the biological detection chip in the present invention.

### Example 3 Animal experiment of African swine fever detection chip

In this example, different African swine fever virus strains were used for challenge experiments in pigs, and various test samples (e.g., blood samples or rectal swab samples) were collected to evaluate the detection capability of the African swine fever detection chip described in the present invention. The results were simultaneously compared with those obtained using real-time PCR (qPCR) as a detection method.

### Materials and methods

Pigs were divided into challenge group and contact group. The challenge group administered low-virulence virus strain (ASFV p72 genotype I), high-virulence virus strain (ASFV p72 genotype II), or recombinant virus strain (ASFV p72 genotype I+ASFV p72 genotype II) for challenge experiments at day 0. The administered concentration of African swine fever virus was 10³ HAD₅₀ in a total volume of 1 mL. The pigs in the contact group were not directly challenged but were housed in the same area as the challenge group to observe the transmission of African swine fever virus. Specifically, the low-virulence strain was administered via intramuscular injection, while the high-virulence and recombinant strains were administered via intranasal injection. The experimental design and the number of pigs per group are summarized in Table 1 below.

**Table. 1 Experimental group design (number of pigs)**

| | challenge group | contact group |
|---|---|---|
| low-virulence virus strain | 3 pigs | 3 pigs |
| high-virulence virus strain | 3 pigs | 3 pigs |
| recombinant virus strain | 3 pigs | 3 pigs |

After the challenge experiments, the body temperature of each pig was measured daily. Samples including oral fluids, rectal swabs, and nasal swabs were collected from each pig. Additionally, blood samples were drawn every two days. These sample collections were conducted for a total of 14 days. Each sample was analyzed using the African swine fever detection chip described in the present invention and simultaneously analyzed using real-time quantitative PCR (qPCR). This example recorded the day when each sample was first detected as positive. For samples from pigs exposed to the same virus strain, the average detection day was calculated to determine on which post-challenge day African swine fever virus could be detected. This enabled a comparison of the detection effectiveness and sensitivity between the chip-based detection method and qPCR, while also observing the patterns of virus infection.

The collected samples in this example were processed using the AttoSense FET Biosensor kit. The pre-treatment steps for the samples are as follows:
Liquid Samples: Take 20 µL of the sample (e.g., whole blood, serum, oral fluid, or nasal fluid) and mix it with 80 µL of lysis buffer. Allow the mixture to stand for 10 minutes.
Swab Samples: Insert the swab (used for nasal, oral, or fecal sampling) into 160 µL of lysis buffer. Rotate the swab 10 times to mix thoroughly, then let it stand for 10 minutes.
After pre-treatment, 20 µL of the processed sample was mixed with dilution buffer and set aside for further use.

This example utilizes quantitative real-time polymerase chain reaction (qPCR) for detection. The nucleotide sequence of the forward primer used in the qPCR reaction is shown as SEQ ID NO: 2, and the nucleotide sequence of the reverse primer is shown as SEQ ID NO: 3. The probe is a TaqMan^{®} probe with a 5' fluorescein amidite (FAM) label and a 3' tetramethylrhodamine (TAM) label, and its nucleotide sequence is shown as SEQ ID NO: 4. The reaction mixture contains 5 µL of 4X TaqMan Fast Virus 1-Step Master Mix (purchased from Biosystems^{™}), 10 µM of primer pairs, 10 µM of the probe, and 5 µL of the test sample, for a total reaction volume of 20 µL. The optimized real-time qPCR reaction conditions are 50°C for 5 minutes, 95°C for 20 seconds, followed by 40 cycles of amplification (95°C for 3 seconds, 58°C for 30 seconds). The real-time qPCR reactions were performed on a LightCycle^{™} 96 system (purchased from Roche, Switzerland), and the data were analyzed using its software.

The chip detection method in this example refers to the method described in Example 2. In this example, the biological detection chip described in the present invention is installed in a handheld detection device, which can be directly connected to a mobile phone or computer for the rapid transmission of detection data to the analysis device.

### Results

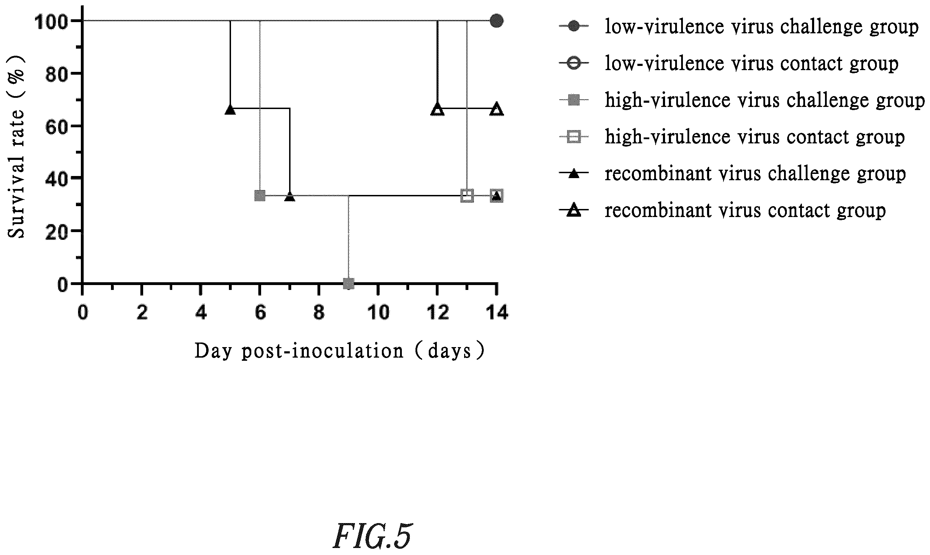

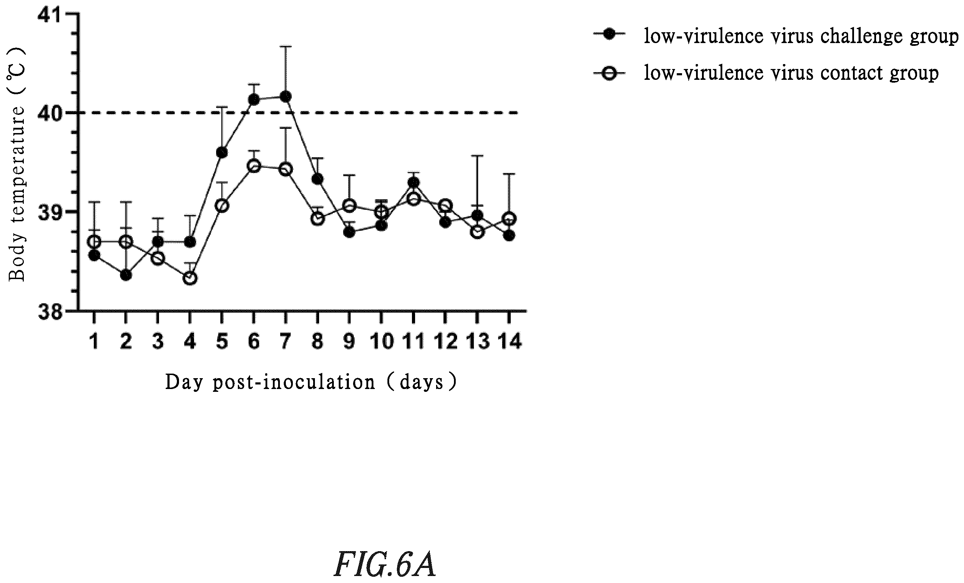

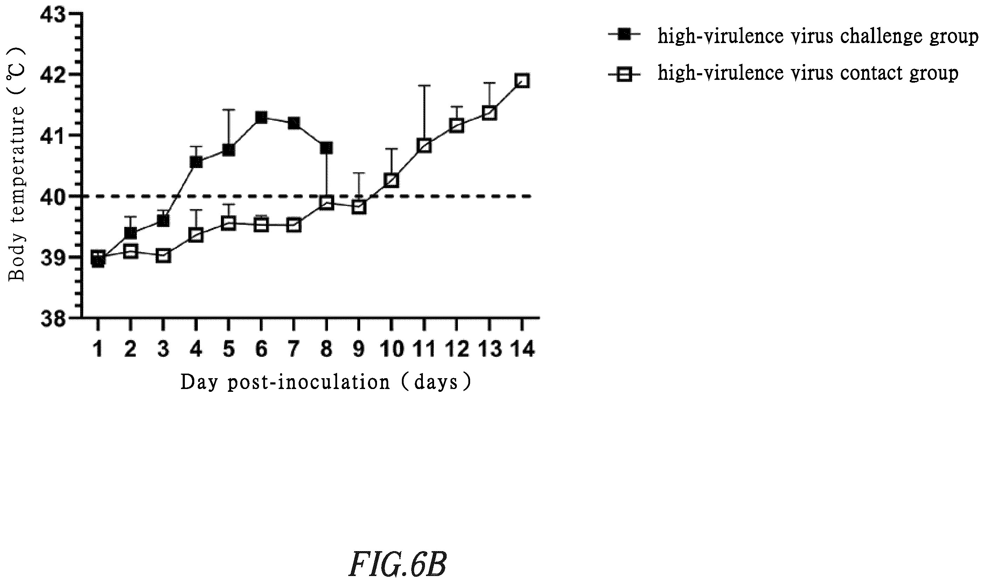

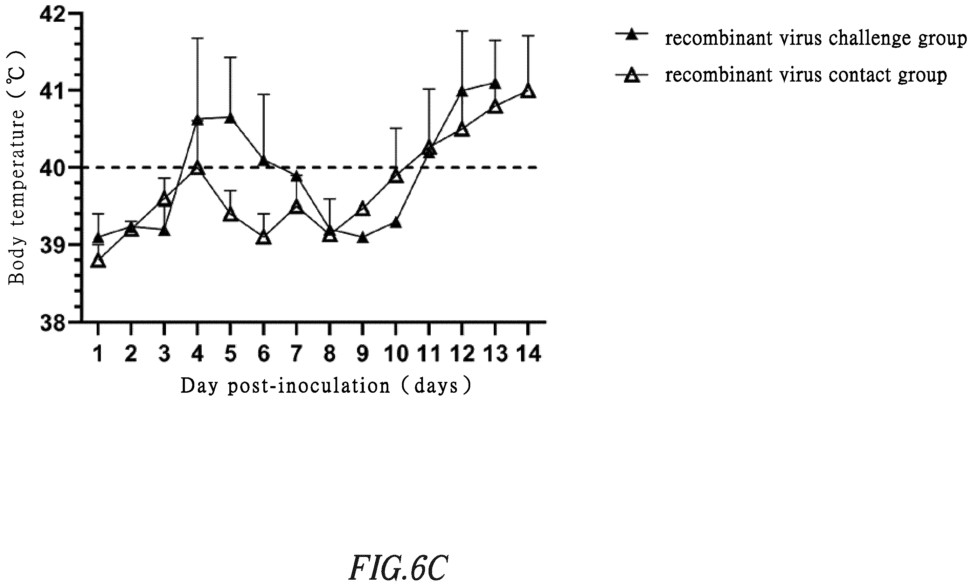

As shown in FIG. 5, which shows the survival rate of pigs over 14 days in this example. The survival rates of pigs in the high-virulence virus challenge group and the recombinant virus challenge group were notably low. In particular, all pigs in the high-virulence virus challenge group died on the 9th day. Furthermore, pigs in the high-virulence virus contact group and the recombinant virus contact group also exhibited deaths in the later stages of the experiment, indicating that unchallenged pigs became infected with the African swine fever virus after contact with virus challenged pigs, ultimately leading to death. Additionally, please refer to FIGs. 6A, 6B, and 6C, which illustrate the average daily body temperatures of pigs in each group. When the body temperature of a pig exceeds 40°C, it is considered to indicate fever. The results showed that pigs in the low-virulence virus challenge group, the high-virulence virus challenge group, and the recombinant virus challenge group exhibited fever around days 4-6. Fever was also observed in the contact groups, especially after the 10th day in the high-virulence virus contact group and the recombinant virus contact group, indicating that unchallenged pigs were infected.

### A. Blood Sample Detection

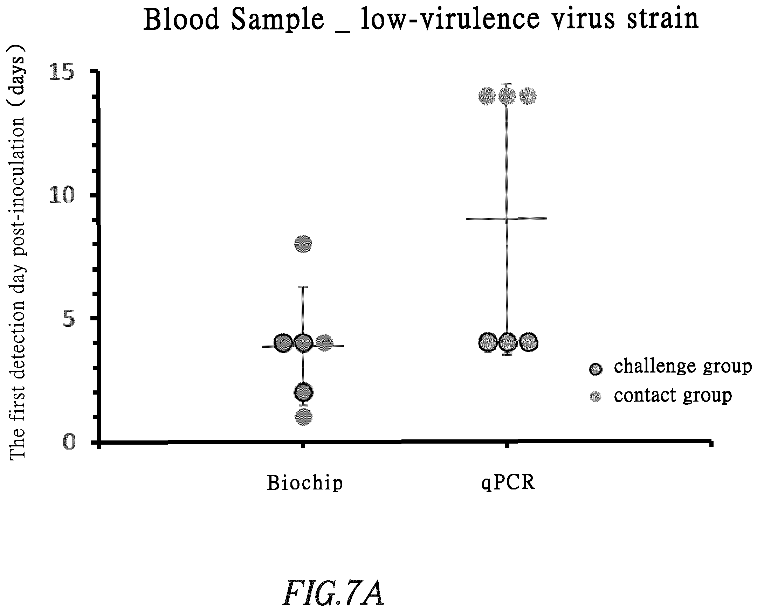

Please refer to FIG. 7A. Using the biological detection chip described in the present invention, the blood samples from the low-virulence virus challenge group detected African swine fever virus on days 2, 4, and 4, while the blood samples from the low-virulence virus contact group detected the virus on days 1, 4, and 8, with an average detection time of 3.83 days after infection. In contrast, using qPCR for detection, the blood samples from the low-virulence virus challenge group detected African swine fever virus on days 4, 4, and 4, while the blood samples from the low-virulence virus contact group detected the virus on days 14, 14, and 14, with an average detection time of 9 days after infection.

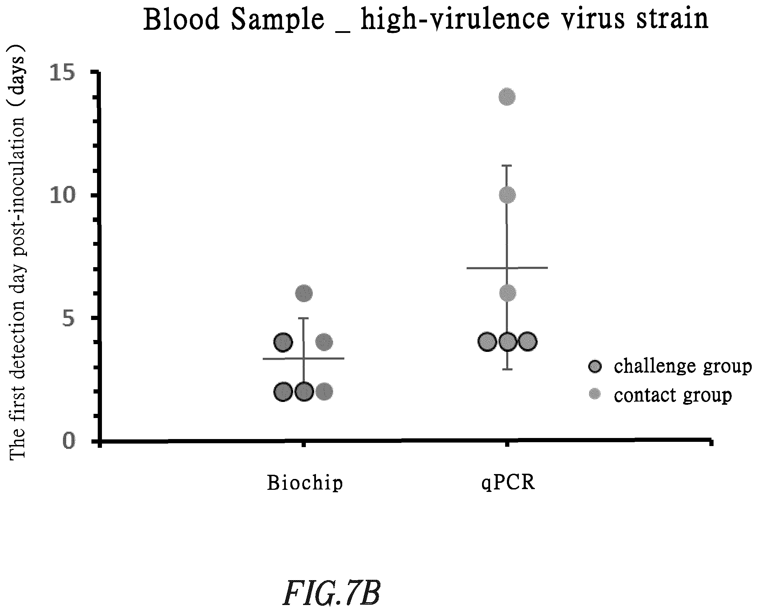

Please refer to FIG. 7B. Using the biological detection chip described in the present invention, the blood samples from the high-virulence virus challenge group detected African swine fever virus on days 2, 2, and 4, while the blood samples from the high-virulence virus contact group detected the virus on days 2, 4, and 6, with an average detection time of 3.33 days after infection. In contrast, using qPCR for detection, the blood samples from the high-virulence virus challenge group detected African swine fever virus on days 4, 4, and 4, while the blood samples from the high-virulence virus contact group detected the virus on days 6, 10, and 14, with an average detection time of 7 days after infection.

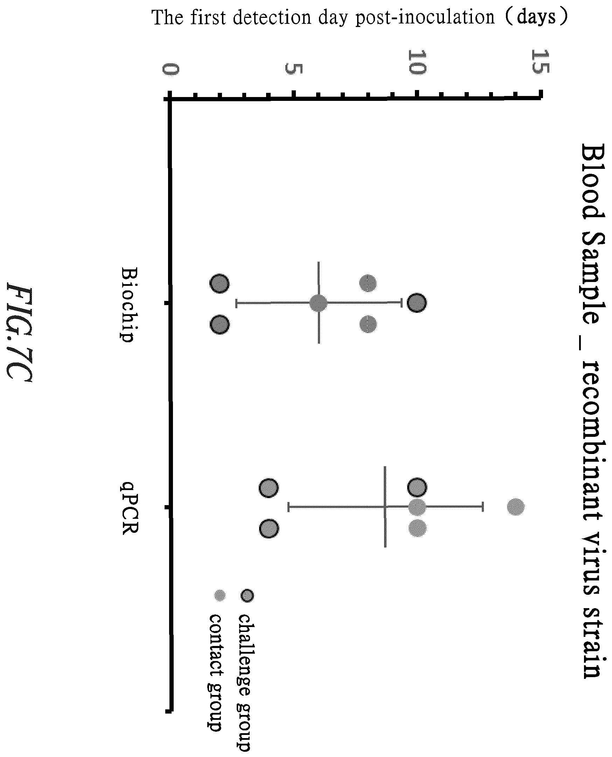

Please refer to FIG. 7C. Using the biological detection chip described in the present invention, the blood samples from the recombinant virus challenge group detected African swine fever virus on days 2, 2, and 10, while the blood samples from the recombinant virus contact group detected the virus on days 6, 8, and 8, with an average detection time of 6 days after infection. In contrast, using qPCR for detection, the blood samples from the recombinant virus challenge group detected African swine fever virus on days 4, 4, and 10, while the blood samples from the recombinant virus contact group detected the virus on days 10, 10, and 14, with an average detection time of 8.67 days after infection.

The detection results of blood samples on each day are shown in Tables 2 to 4. In the tables, the denominator represents the total number of pigs, while the numerator represents the number of pigs that tested positive, where positive indicates that African swine fever virus was detected in the sample from the pig. For example, 0/3 means that out of 3 pigs, 0 tested positive; 1/3 means that out of 3 pigs, 1 tested positive; 2/3 means that out of 3 pigs, 2 tested positive; and 3/3 means that out of 3 pigs, all tested positive. Additionally, samples from deceased pigs are considered positive samples.

The blood sample detection of low-virulence virus strains is shown in Table 2 below. When the low-virulence virus contact group was tested by qPCR, African swine fever virus could not be detected. The reason is that the virus concentration is low. In comparison, the infection of pigs can be detected through biochip detection, showing that the biological detection chip described in the present invention has higher sensitivity.

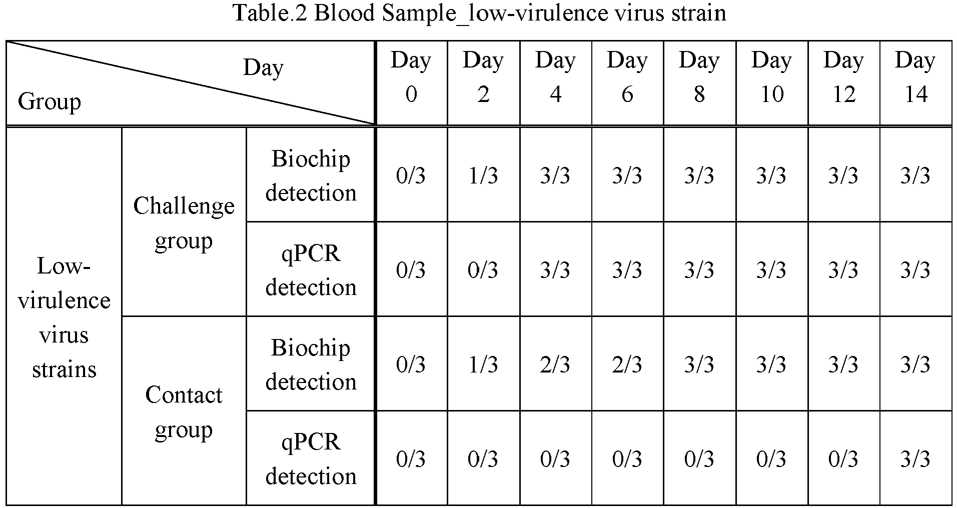

The blood sample detection of high-virulence virus strains is shown in Table 3 below. The result shows that the efficacy of the biochip detection is better than PCR detection, especially in the high-virulence virus strain contact group. The result indicates that the African swine fever detection chip described in the present invention has higher sensitivity than qPCR.

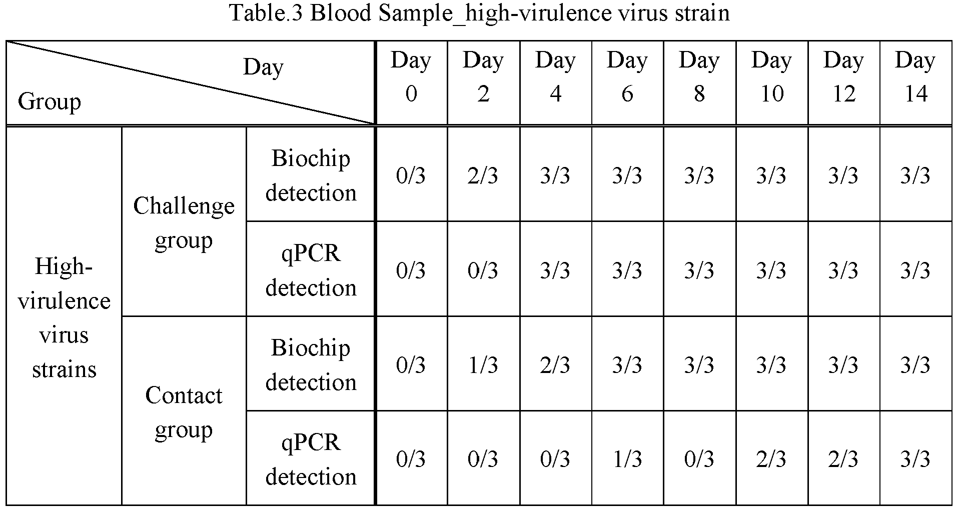

The blood sample detection of the recombinant virus strain is shown in Table 4 below. The result shows that the efficacy of the biochip detection is better than qPCR detection, especially in the recombinant virus strain contact group. The result indicates that the African swine fever detection chip described in the present invention exhibits higher efficiency and sensitivity than qPCR detection.

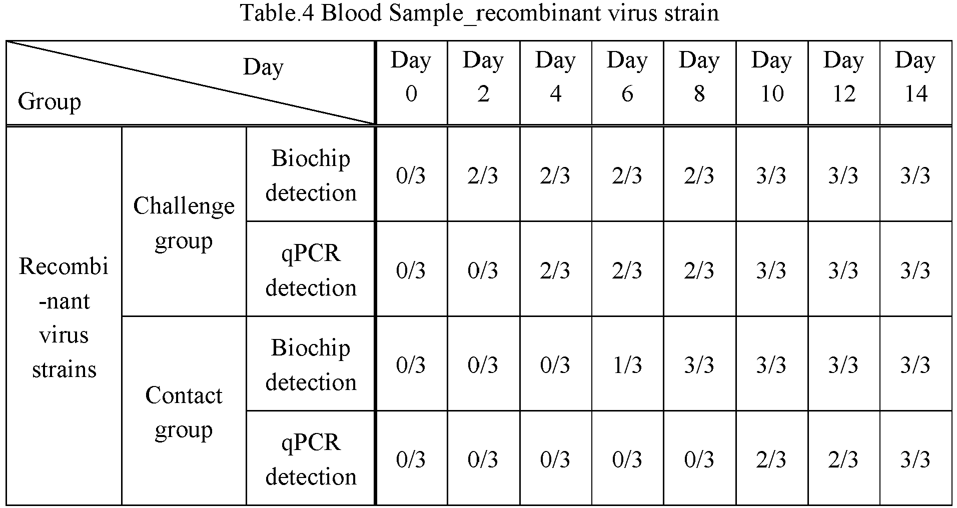

A comprehensive review of the blood sample detection results shows that using the African swine fever detection chip described in the present invention, the virus can be detected in pigs' blood samples within an average of 4.386 days after infection. In contrast, qPCR detection must increase the concentration of virus in pigs' body in order to be effectively detected, with an average of 8.223 days. Continuous monitoring further demonstrates the stability of the chip detection method. The results indicate that the biological detection chip described in the present invention is applicable to blood samples, offers higher sensitivity, and is suitable for detecting different African swine fever virus strains.

### B. Nasal Swab Sample Detection

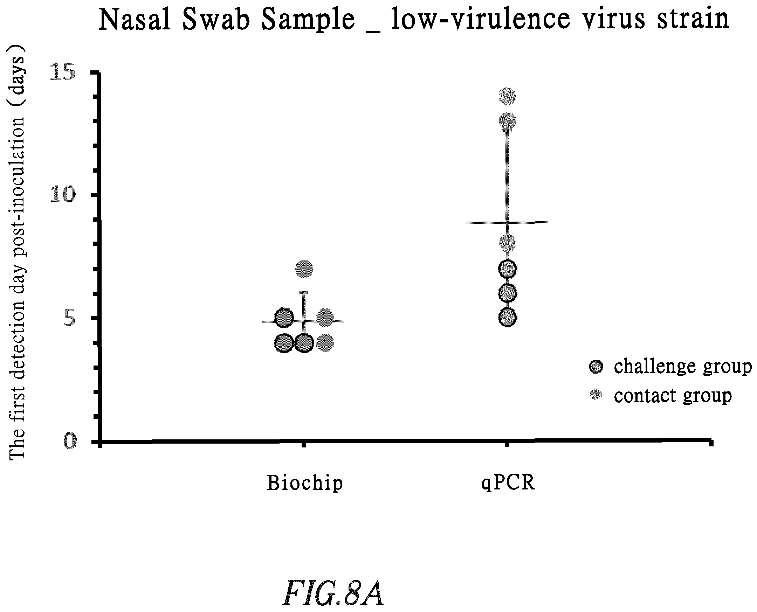

Please refer to FIG. 8A. Using the biological chip described in the present invention, nasal swab samples from the low-virulence virus challenge group detected African swine fever virus on days 4, 4, and 5, while the nasal swab samples from the low-virulence virus contact group detected the virus on days 4, 5, and 7, with an average detection time of 4.83 days after infection. In contrast, using qPCR for detection, the nasal swab samples from the low-virulence virus challenge group detected African swine fever virus on days 5, 6, and 7, while the nasal swab samples from the low-virulence virus contact group detected the virus on days 8, 13, and 14, with an average detection time of 8.83 days after infection.

The results indicate that qPCR struggles to detect African swine fever virus in the low-virulence contact group during the early stages of infection due to low viral concentrations, typically only becoming detectable after approximately day 8. In comparison, the biochip detection method effectively identifies the infection even during the early stages when the viral concentration is low, demonstrating the superior sensitivity of the biological detection chip described in the present invention.

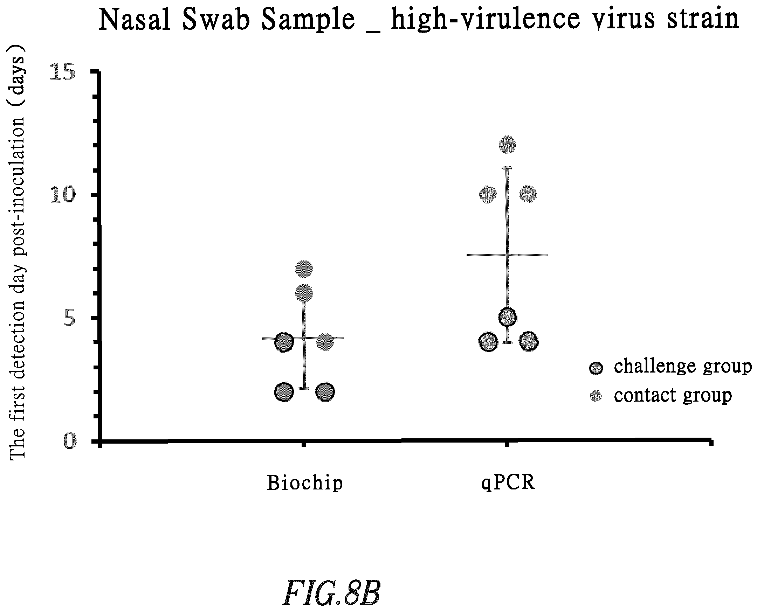

Please refer to FIG. 8B. Using the biological detection chip described in the present invention, nasal swab samples from the high-virulence virus challenge group detected African swine fever virus on days 2, 2, and 4, while the nasal swab samples from the high-virulence virus contact group detected the virus on days 4, 6, and 7, with an average detection time of 4.17 days after infection. In contrast, using qPCR for detection, the nasal swab samples from the high-virulence virus challenge group detected African swine fever virus on days 4, 4, and 5, while the nasal swab samples from the high-virulence virus contact group detected the virus on days 10, 10, and 12, with an average detection time of 7.5 days after infection.

The results show that the efficacy of the biological detection chip described in the present invention is better than that of qPCR detection, especially in the high-virulence virus contact group. qPCR detection can only detect the ASFV in the late stage after infection when the virus concentration is higher. In comparison, the biological detection chip can detect ASFV in the early stage after infection, indicating that the biological detection chip described in the present invention has higher sensitivity.

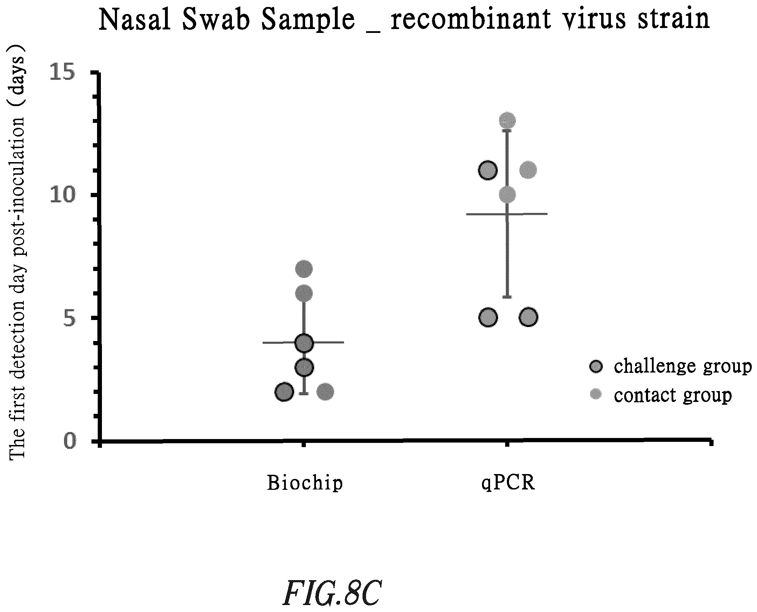

Please refer to FIG. 8C. Using the biological detection chip described in the present invention, nasal swab samples from the recombinant virus challenge group detected African swine fever virus on days 2, 3, and 4, while the nasal swab samples from the recombinant virus contact group detected the virus on days 2, 6, and 7, with an average detection time of 4 days after infection. In contrast, using qPCR for detection, the nasal swab samples from the recombinant virus challenge group detected African swine fever virus on days 5, 5, and 11, while the nasal swab samples from the recombinant virus contact group detected the virus on days 10, 11, and 13, with an average detection time of 9.17 days after infection.

The results show that the efficacy of the biological detection chip described in the present invention is better than that of qPCR detection, especially in the recombinant virus contact group. qPCR detection can only detect the ASFV in the late stage after infection when the virus concentration is higher. In comparison, the biological detection chip can detect ASFV in the early stage after infection, indicating that the biological detection chip described in the present invention has higher sensitivity.

In conclusion of the detection results of the nasal swab samples, using the biological detection chip described in the present invention, it took an average of only 4.33 days from the day of infection to detect the presence of African swine fever virus in pigs. In contrast, qPCR detection requires a higher viral load to effectively detect the virus, averaging 8.5 days. Moreover, qPCR requires samples to be transported back to a laboratory for analysis using real-time qPCR equipment, which involves more cumbersome procedures. The results demonstrate that the biological detection chip described in the present invention can be effectively used for nasal swab samples. The biological detection chip exhibits higher sensitivity and is suitable for detecting various strains of African swine fever virus.

### C. Oral Fluid Sample Detection

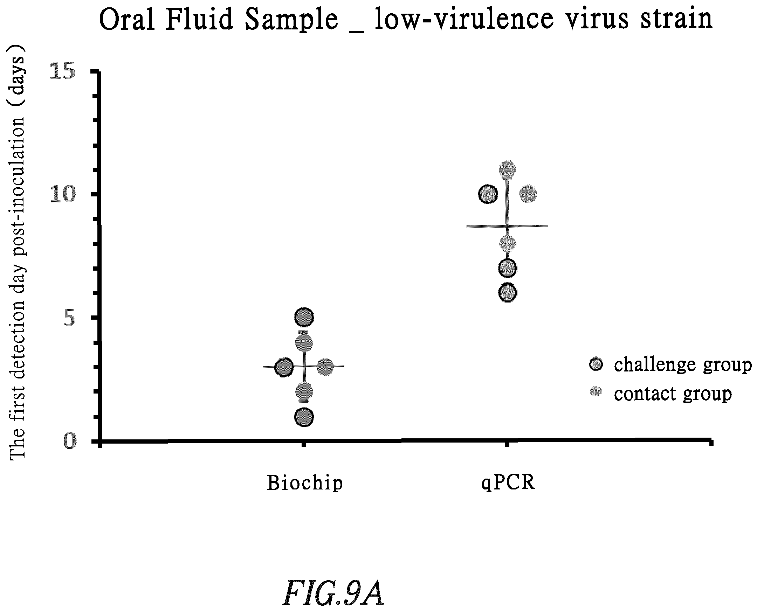

Please refer to FIG. 9A. Using the biological chip described in the present invention, oral fluid samples from the low-virulence virus challenge group detected African swine fever virus on days 1, 3, and 5, while the oral fluid samples from the low-virulence virus contact group detected the virus on days 2, 3, and 4, with an average detection time of 3 days after infection. In contrast, using qPCR for detection, the oral fluid samples from the low-virulence virus challenge group detected African swine fever virus on days 6, 7, and 10, while the oral fluid samples from the low-virulence virus contact group detected the virus on days 8, 10, and 11, with an average detection time of 8.67 days after infection.

The results indicate that qPCR struggles to detect African swine fever virus in the low-virulence contact group during the early stages of infection due to low viral concentrations, typically only becoming detectable after approximately day 9. In comparison, the biochip detection method effectively identifies the infection even during the early stages when the viral concentration is low, demonstrating the superior sensitivity of the biological detection chip described in the present invention.

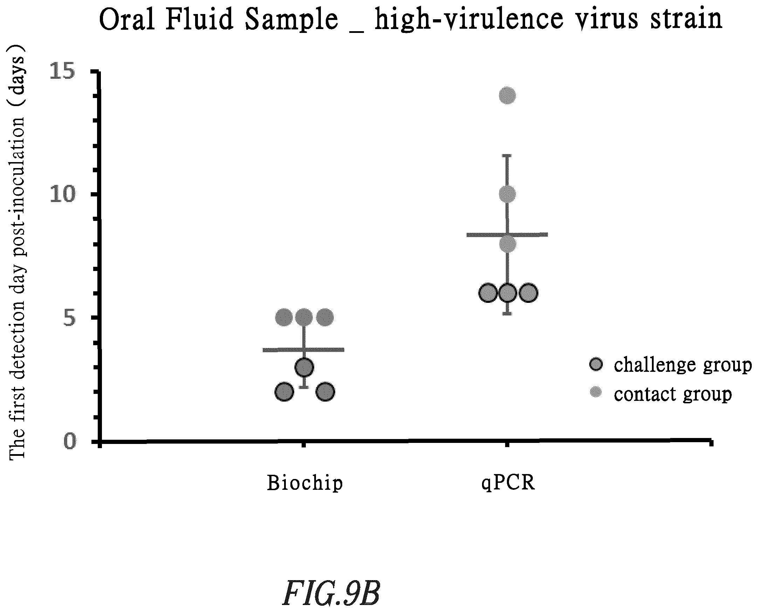

Please refer to FIG. 9B. Using the biological detection chip described in the present invention, oral fluid samples from the high-virulence virus challenge group detected African swine fever virus on days 2, 2, and 3, while the oral fluid samples from the high-virulence virus contact group detected the virus on days 5, 5, and 5, with an average detection time of 3.6 days after infection. In contrast, using qPCR for detection, the oral fluid samples from the high-virulence virus challenge group detected African swine fever virus on days 6, 6, and 6, while the oral fluid samples from the high-virulence virus contact group detected the virus on days 8, 10, and 14, with an average detection time of 8.3 days after infection.

The results show that the efficacy of the biological detection chip described in the present invention is better than that of qPCR detection. Regardless of whether they are in the challenge group or contact group of high-virulence virus strains, qPCR testing can only detect the ASFV in the late stage after infection when the virus concentration is higher. In comparison, the biological detection chip can detect ASFV in the early stage after infection, indicating that the biological detection chip described in the present invention has higher sensitivity.

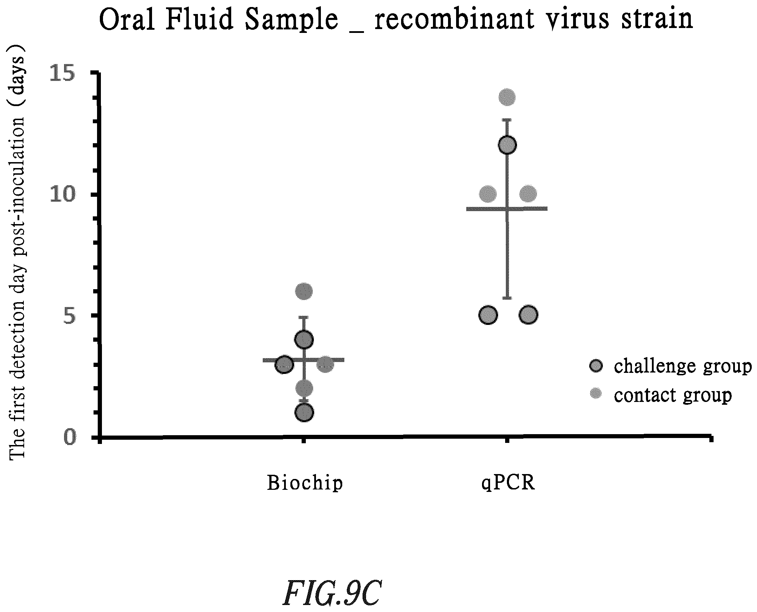

Please refer to FIG. 9C. Using the biological detection chip described in the present invention, oral fluid samples from the recombinant virus challenge group detected African swine fever virus on days 1, 3, and 4, while the nasal swab samples from the recombinant virus contact group detected the virus on days 2, 3, and 6, with an average detection time of 3.17 days after infection. In contrast, using qPCR for detection, the nasal swab samples from the recombinant virus challenge group detected African swine fever virus on days 5, 5, and 12, while the nasal swab samples from the recombinant virus contact group detected the virus on days 10, 10, and 14, with an average detection time of 9.33 days after infection.

The results show that the efficacy of the biological detection chip described in the present invention is better than that of qPCR detection. Regardless of whether they are in the challenge group or contact group of recombinant virus strains, qPCR testing can only detect the ASFV in the late stage after infection when the virus concentration is higher. In comparison, the biological detection chip can detect ASFV in the early stage after infection, indicating that the biological detection chip described in the present invention has higher sensitivity.

In conclusion of the detection results of the oral fluid samples, using the biological detection chip described in the present invention took an average of 3.256 days from the day of infection to detect the presence of African swine fever virus in pigs. In contrast, qPCR detection requires a higher viral load to effectively detect the virus, averaging 8.766 days. Moreover, qPCR requires samples to be transported back to a laboratory for analysis using real-time qPCR equipment, which involves more cumbersome procedures. The results demonstrate that the biological detection chip described in the present invention can be effectively used for oral fluid samples. The biological detection chip exhibits higher sensitivity and is suitable for detecting various strains of African swine fever virus.

### D. Rectal Swab Sample Detection

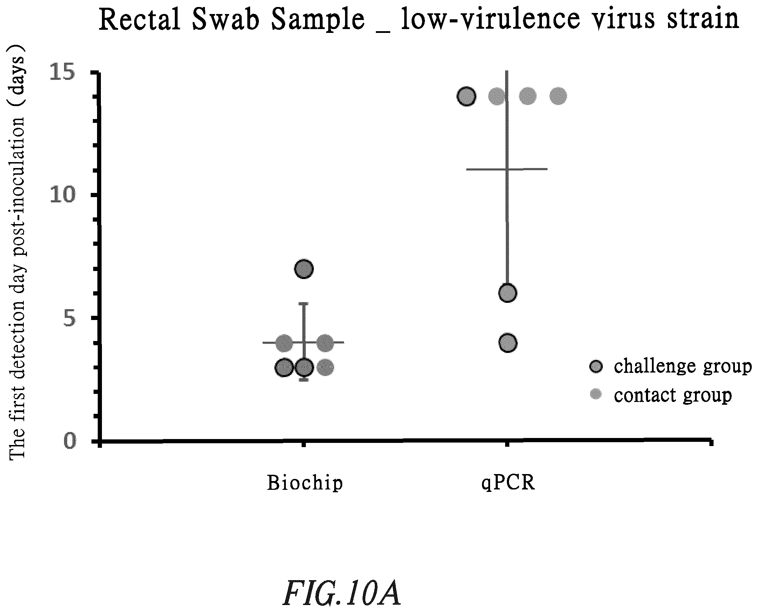

Please refer to FIG. 10A. Using the biological chip described in the present invention, rectal swab samples from the low-virulence virus challenge group detected African swine fever virus on days 3, 3, and 7, while the rectal swab samples from the low-virulence virus contact group detected the virus on days 3, 4, and 4, with an average detection time of 4 days after infection. In contrast, using qPCR for detection, the rectal swab samples from the low-virulence virus challenge group detected African swine fever virus on days 4, 6, and 14, while the rectal swab samples from the low-virulence virus contact group detected the virus on days 14, 14, and 14, with an average detection time of 11 days after infection.

The results indicate that qPCR struggles to detect African swine fever virus in the low-virulence contact group during the early stages of infection due to low viral concentrations. In comparison, the biochip detection method effectively identifies the infection even during the early stages when the viral concentration is low, demonstrating the superior sensitivity of the biological detection chip described in the present invention.

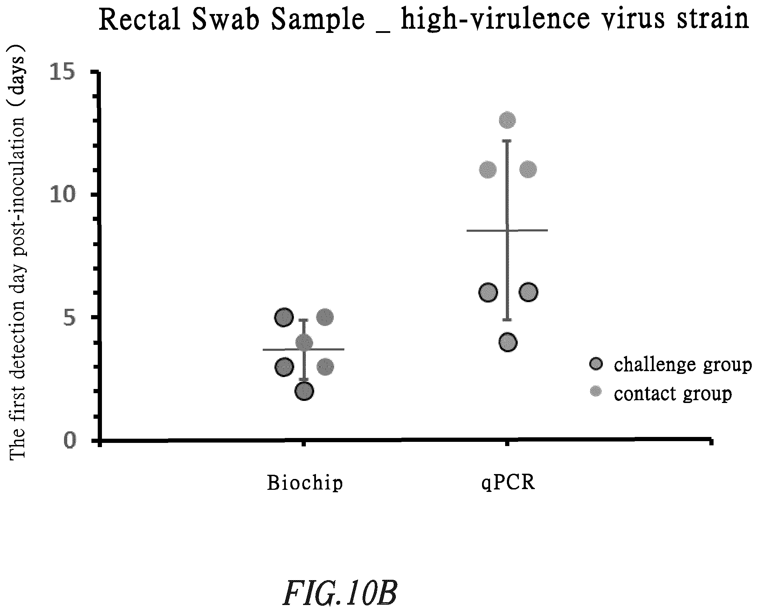

Please refer to FIG. 10B. Using the biological detection chip described in the present invention, rectal swab samples from the high-virulence virus challenge group detected African swine fever virus on days 2, 3, and 5, while the rectal swab samples from the high-virulence virus contact group detected the virus on days 3, 4, and 5, with an average detection time of 3.67 days after infection. In contrast, using qPCR for detection, the rectal swab samples from the high-virulence virus challenge group detected African swine fever virus on days 4, 6, and 6, while the rectal swab samples from the high-virulence virus contact group detected the virus on days 11, 11, and 13, with an average detection time of 8.5 days after infection.

The results show that the efficacy of the biological detection chip described in the present invention is better than that of qPCR detection, especially in the high-virulence virus contact group. qPCR testing can only detect the ASFV in the late stage after infection when the virus concentration is higher. In comparison, the biological detection chip can detect ASFV in the early stage after infection, indicating that the biological detection chip described in the present invention has higher sensitivity.

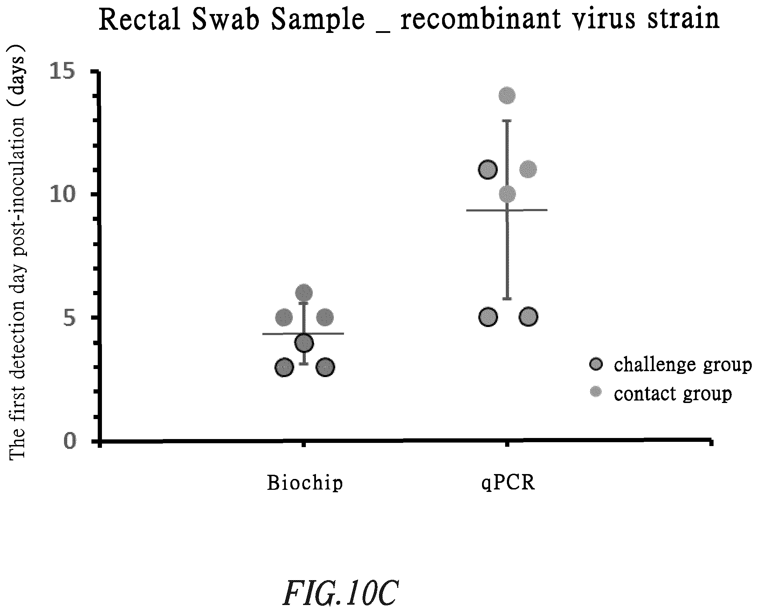

Please refer to FIG. 10C. Using the biological detection chip described in the present invention, rectal swab samples from the recombinant virus challenge group detected African swine fever virus on days 3, 3, and 4, while the rectal swab samples from the recombinant virus contact group detected the virus on days 5, 5, and 6, with an average detection time of 4.33 days after infection. In contrast, using qPCR for detection, the rectal swab samples from the recombinant virus challenge group detected African swine fever virus on days 5, 5, and 11, while the rectal swab samples from the recombinant virus contact group detected the virus on days 10, 11, and 14, with an average detection time of 9.33 days after infection.

The results show that the efficacy of the biological detection chip described in the present invention is better than that of qPCR detection. Regardless of whether they are in the challenge group or contact group of recombinant virus strains, qPCR testing can only detect the ASFV in the late stage after infection when the virus concentration is higher. In comparison, the biological detection chip can detect ASFV in the early stage after infection, indicating that the biological detection chip described in the present invention has higher sensitivity.

In conclusion of the detection results of the rectal swab samples, using the biological detection chip described in the present invention took an average of 4 days from the day of infection to detect the presence of African swine fever virus in pigs. In contrast, qPCR detection requires a higher viral load to effectively detect the virus, averaging 9.61 days. Moreover, qPCR requires samples to be transported back to a laboratory for analysis using real-time qPCR equipment, which involves more cumbersome procedures. The results demonstrate that the biological detection chip described in the present invention can be effectively used for rectal swab samples. The biological detection chip exhibits higher sensitivity and is suitable for detecting various strains of African swine fever virus.

In summary, the biological detection chip described in the present invention can effectively detect low concentration of the virus, demonstrating exceptionally high sensitivity. It enables rapid detection during the early stage of ASFV infection, which is crucial for controlling the virus outbreaks. Additionally, the detection chip offers high stability in detection. Moreover, the biological detection chip is applicable to various types of samples, and the biological probes described in the present invention can effectively detect different variants of the African swine fever virus. The operation is simple and fast, making it highly advantageous for industrial applications. This technology holds broad application potential in the prevention and control of African swine fever virus outbreaks.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising a plurality of biological probes, wherein the biological probes comprise a nucleotide sequence of SEQ ID NO: 1;
wherein the biological detection layers of the plurality of transistors form a biological detection area on the surface of the biological detection chip.

2. The biological detection chip according to claim 1, wherein the biological detection layer is generated through a surface modification process on the surface of the extending gate.

3. The biological detection chip according to claim 2, wherein the surface modification process is selected from the group consisting of 3-aminopropyltrimethoxysilane-Glutaraldehyde (APTES-GA) modification method, 3-aminopropyltrimethoxysilane-N-hydroxysuccinimide (APTES-NHS) modification method, 3-aminopropyltrimethoxysilane-Biotin (APTES-Biotin) modification method, and 3-glycidoxypropyltrimethoxysilane (GPTMS) modification method.

4. The biological detection chip according to any one of claims 1 to 3, wherein the biological detection area comprises a microfluidic channel or a sample reservoir.

5. The biological detection chip according to any one of claims 1 to 4, wherein the biological detection chip is set on a handheld detector.

6. A method for detecting African swine fever virus, comprising the following steps:
providing a biological detection chip of claim 1;
placing a blank sample in the biological detection area, and measuring a gate voltage and a drain current of the plurality of transistors to establish a standard curve;
placing a test sample in the biological detection area for a predetermined time;
washing the biological detection area by the blank sample;
measuring the gate voltage and the drain current of the plurality of transistors to establish a measurement curve; and
comparing the measurement curve with the standard curve to determine whether the test sample contains African swine fever virus.

7. The detection method according to claim 6, wherein the test sample is derived from a pig.

8. The detection method according to claim 6 or 7, wherein the test sample is a liquid sample.

9. The detection method according to claim 8, wherein the liquid sample comprises a whole blood sample, a serum sample, an oral fluid sample or a urine sample.

10. The detection method according to claim 6 or 7, wherein the test sample is a swab sample.

11. The detection method according to claim 10, wherein the swab sample comprises a nasal swab sample, an oral swab sample or a rectal swab sample.

12. A biological probe, comprising a nucleotide sequence of SEQ ID NO: 1, wherein the biological probe is used for detecting African swine fever virus.

13. The biological probe according to claim 12, wherein the nucleotide sequence comprising at least 90% sequence identity to SEQ ID NO: 1.
